Europäisches Patentamt

(19) European Patent Office

Office européen des brevets

(11) Veröffentlichungsnummer: **0 093 927**
**B1**

(12) **EUROPÄISCHE PATENTSCHRIFT**

(45) Veröffentlichungstag der Patentschrift:
07.01.87

(51) Int. Cl.⁴: **A 61 B 5/00, A 61 B 1/00,**
**A 61 B 5/14, G 01 N 21/85**

(21) Anmeldenummer: 83103989.6

(22) Anmeldetag: 23.04.83

(54) **Gerät zur Spektrenmessung in der Blutbahn.**

(30) Priorität: 29.04.82 DE 3215879

(43) Veröffentlichungstag der Anmeldung:
16.11.83 Patentblatt 83/46

(45) Bekanntmachung des Hinweises auf die Patenterteilung:
07.01.87 Patentblatt 87/2

(84) Benannte Vertragsstaaten:
CH DE FR GB IT LI NL SE

(56) Entgegenhaltungen:
DE - A - 2 726 606
DE - A - 2 939 231
GB - A - 1 128 226
US - A - 3 624 816
US - A - 3 674 013
US - A - 3 807 390
US - A - 4 213 462

IEEE TRANSACTIONS ON BIOMEDICAL ENGINEERING,
Band BME-26, Nr. 7, Juli 1979, Seiten 416-422, IEEE, New
York, USA
IEEE TRANSACTIONS ON BIOMEDICAL ENGINEERING,
Band BME-26, Nr. 7, Juli 1979, Seiten 416-422, IEEE, New
York, USA. R. J. VOLZ et al.: "A neonatal fiberoptic
probe for oximetry and dye curves"

(73) Patentinhaber: **Firma Carl Zeiss, D-7920 Heidenheim
(Brenz) (DE)**

(72) Erfinder: **Mächler, Meinrad, Sulzgasse 2,
D-7090 Ellwangen (DE)**
Erfinder: **Mächler, Sibylle, Dr., Sulzgasse 2,
D-7090 Ellwangen (DE)**
Erfinder: **Sachse, Richard, Ing. grad., Ulmenweg 21,
D-7923 Königsbronn (SE)**

## Beschreibung

Die Erfindung betrifft ein Gerät zur Spektrenmessung mit einem Diodenzeilenspektrometer, einer Beleuchtungseinrichtung und einer y-förmigen Lichtleitersonde, deren erster Zweig mit der Beleuchtungseinrichtung und deren zweiter Zweig mit dem Diodenzeilenspektrometer verbunden ist, so dass einem Eintrittsspalt des Diodenzeilenspektrometers das vom untersuchten Objekt veränderte Licht zugeführt ist, und deren Stamm in einem Messkopf endet.

Eine photometrische Messung in der Blutbahn hat z.B. Bedeutung für die kontinuierliche Messung des Sauerstoffgehaltes von Blut. Dieser ist für die Anästhesie von besonderer Wichtigkeit, da eine Sauerstoffunterversorgung während einer Narkose zu schweren irreparablen Schäden beim Patienten führen kann. Die einzige bisher in der Routine verbreitete Messmethode ist eine polarographische Methode («Oxymeter» von Dräger AG und Hellige GmbH), die seit 1975 eingeführt ist und auf einen Vorschlag von Huch und Lübbers zurückgeht (Arch. Gynäkol. 207, 443, 1969). Die Methode hat die Nachteile einer «Einlaufzeit» von 15 bis 20 min, einer systembedingten Verzögerungszeit von etwa 15 sec, der Notwendigkeit der Kalibrierung vor jeder Messung und einer ebenfalls systembedingten Drift von 10–15% über einige Stunden. Ausserdem besteht die Notwendigkeit gelegentlich durchzuführender arterieller Blutgasanalysen zur Funktionskontrolle der Elektrode.

Aus der DE-OS 2 726 606 ist ein Spektralphotometer zur Messung an Geweboberflächen bekannt, bei dem das Spektrum auf einem Oszillographen sichtbar gemacht wird. Das Photometer arbeitet mit einer y-förmigen Lichtleitersonde, deren erster Zweig mit einer Beleuchtungseinrichtung verbunden ist und deren Stamm in einem Messkopf endet, welcher auf die Geweboberfläche aufgesetzt wird. Das von dieser veränderte Licht wird über den zweiten Zweig der Lichtleitersonde dem Eintrittsspalt eines Monochromators zugeführt, der mit einer Diodenzeile (dort als Photozellenarray bezeichnet) ausgerüstet ist.

In der Zusatz-OS 2 815 074 zu der erwähnten Anmeldung hat der Sichtschirm des Oszillographen an den charakteristischen Punkten des Hämoglobinspektrums liegende Marken zur Erleichterung der Auswertung.

Es ist bekannt, z.B. aus Documenta Geigy, wissenschaftliche Tabellen, 6. Auflage, 1960, dass sich die Absorptionsmaxima der Spektren von Hämoglobin, der reduzierten Form, und Oxyhämoglobin, der oxydierten Form, in charakteristischer Weise verschieben: das langwellige Absorptionsmaximum von 560 nach 577 nm, das kurzwellige von 430 nach 412 nm. Der Abstand der beiden Maxima ändert sich also als Funktion des Sauerstoffgehalts um 35 nm, und zwar unabhängig vom Absolutwert der Absorption. Diese Unabhängigkeit vom Absolutwert der Absorption ist die wesentlichste Voraussetzung für die Durchführbarkeit einer exakten Sauerstoffgehaltsbestimmung. Es ist bekannt und einleuchtend, dass der Sauerstoffgehalt «in vivo» nur unter Ausschluss von Luftsauerstoff bestimmt werden kann. Es ist weiterhin bekannt, dass die Zahl der Erythrocyten, der Träger des Hämoglobins, rund 5 Millionen pro Kubikmillimeter beträgt (40 bis 45% des Blutvolumens), bei einem mittleren Durchmesser von 7,5 μm. Das bedeutet, das wegen der hohen Streukoeffizienten eine photometrische Messung in Transmission unmöglich wäre. Andererseits ist aber die Opazität noch so gross, dass auch eine echte Reflexionsmessung nicht möglich ist.

Aus einer Veröffentlichung von Curtis C. Johnson (J.o.t. Association f.t. Advancement o. Medical Instrumentation 5, 77, (1971)) ist bekannt, eine Reflexionsmessung in vivo mit einem Lichtleitermesskopf durchzuführen, der in ein Herzkatheter eingeführt wird. Die Messung erfolgt bei zwei Wellenlängen; als Lichtquellen dienen zwei LED, als Empfänger eine Silizum-Photodiode. Die Messung im Herzinneren ist wegen des grossen Durchmessers des Lichtleiters notwendig. Es ist selbstverständlich, dass eine derartige Messung nur in Ausnahmefällen in Frage kommt und nicht für die Routine bei normalen Operationen geeignet ist.

In energetischer Hinsicht ist eine Vorrichtung sehr viel günstiger, die von U. Tutschke (Biomedizinische Technik 21, 279 (1976)) beschrieben ist. Für die Erzeugung des Messlichtes bei zwei verschiedenen Wellenlängen werden ein He-Ne-Laser und ein Impuls-Farbstofflaser verwendet. Die gegenüber herkömmlichen Lichtquellen sehr viel grösseren Lichtintensitäten haben entsprechende Rückstreusignale zur Folge, so dass gegenüber anderen Verfahren mit einem günstigeren Signal-Rausch-Verhältnis gemessen werden kann und/oder Lichtleiter mit kleinerem Durchmesser verwendet werden können, die eine intravasale Messung ermöglichen. Für die medizinische Routine ist eine Messung mit zwei Lasern jedoch zu aufwendig, so dass ein derartiges Gerät nicht die Anforderungen der Praxis erfüllt.

Die Messung bei zwei Wellenlängen kann die spektrale Verschiebung der Absorptionsmaxima nicht direkt erfassen, sondern nur die Intensitäts-Änderung einer charakteristischen Wellenlänge gegenüber einem isobestischen Punkt. Für quantitative Bestimmungen ist es günstiger, im relevanten Bereich den gesamten Spektrenverlauf verfügbar zu haben und damit die Genauigkeit zu verbessern. Ausserdem ist ein Gerät, welches den gesamten Spektrenverlauf misst, universell einsetzbar.

Der vorliegenden Erfindung liegt daher die Aufgabe zugrunde, ein für den medizinischen Routinebetrieb geeignetes Gerät zu schaffen, das die Messung des Streulicht-Spektrums von Blut innerhalb einer peripheren Vene oder Arterie ermöglicht, ohne den freien Durchfluss des Blutes durch das Blutgefäss zu unterbinden.

Die gestellte Aufgabe wird gemäss der Erfindung dadurch gelöst, dass ein in eine Blutbahn einführbarer Teil des Messkopfes der Lichtleitersonde mit einem Durchmesser von maximal 0,5 mm ausgebildet ist, dass jeder der beiden Zweige der Lichtleitersonde durch eine Steckverbindung und ein diese aufnehmendes Fassungsteil mit genau definierter Lage der Lichtleiter mit dem Diodenzeilenspektrometer bzw. mit der Beleuchtungseinrichtung verbunden ist, dass die Endflächen der Lichtfasern in der Steckverbindung zum Diodenzeilenspektrometer den Eintrittsspalt des Diodenzeilenspektrometers bilden, dass das Diodenzeilenspektrometer ein holographisch erzeugtes Beugungsgitter aufweist und ein Öffnungsverhältnis hat, das die Apertur des Lichtleiters ohne Beschnitt aufnimmt.

Durch die Steckverbindungen mit genau definierter Lage der Lichtleiter kann die Lichtleitersonde ausgewechselt werden, ohne dass irgendwelche Justier- oder Befestigungsvorgänge erforderlich sind und ohne dass die Bedienungsperson mit unsterilen Gegenständen in Berührung kommt. Dies ist für den Routinebetrieb ein wesentlicher Vorteil, da die Lichtleitersonde entweder zum Sterilisieren ausgewechselt werden muss oder als sterilisiertes Wegwerfteil für die Einmalbenutzung ausgebildet ist.

Durch das grosse Öffnungsverhältnis des Spektrometers, das die Apertur des Lichtleiters ohne Beschnitt aufnimmt, werden günstige Energieverhältnisse erreicht, obwohl am Messort nur ein kleines Volumenelement erfasst wird, da von dem kleinen Volumenelement die in einen grossen Raumwinkel gestreute Strahlung ausgenutzt wird.

Es ist zweckmässig, die Sonde aus mehreren Lichtleitfasern für die Hinleitung des Lichtes zum Messort und aus mehreren Lichtleitfasern für die Fortleitung des Lichtes vom Messort zusammenzusetzen und sie mit inerten Hüllen zum Schutz gegen Bruch und Blutgerinnung zu umgeben.

In einer besonders vorteilhaften Ausführungsform sind die Lichtleitfasern in der Steckverbindung für das Diodenzeilenspektrometer in Reihe angeordnet.

Am Messkopf können die Lichtleitfasern für die Beleuchtung konzentrisch angeordnet und ringförmig von den Lichtleitfasern für die Messung umgeben sein. Es hat sich jedoch herausgestellt, dass bei der Messung im Blut infolge der starken Streuung auch eine Durchmischung der Lichtleitfasern günstig ist, sofern diese einigermassen statistisch erfolgt.

In einer zweckmässigen Ausführungsform ist die Lichtleitersonde für die Einführung in ein Verweilkatheter ausgebildet. Besonders günstig ist es, in der Lichtleitersonde einen oder mehrere Spülkanäle vorzusehen, damit das Festsetzen von Blutkörperchen am Messkopf verhindert werden kann.

Die Verwendung einer Xenonlampe als Lichtquelle bietet nicht nur einen energetischen Vorteil, sondern auch eine höhere Betriebssicherheit, da eine Xenonlampe am Ende ihrer Lebensdauer sich nicht mehr zünden lässt, aber so gut wie nie während des Betriebes ausgeht.

Weitere Ausgestaltungen der Erfindung gehen aus den Unteransprüchen hervor.

Die Erfindung wird im folgenden anhand der Figuren 1 bis 5 näher erläutert. Dabei zeigen:

Fig. 1 eine schematische Darstellung des Aufbaus des neuen Gerätes,

Fig. 2 die Ausbildung des Kopfes der Lichtleitersonde zusammen mit einem Verweilkatheter,

Fig. 3 ein Beispiel für die Verteilung der Lichtleitfasern für Beleuchtung und Messung am Messkopf,

Fig. 4 ein Ausführungsbeispiel für die Steckverbindungen und

Fig. 5 Ausführungsbeispiele für die Anordnung der Lichtleiter in den Steckverbindungen.

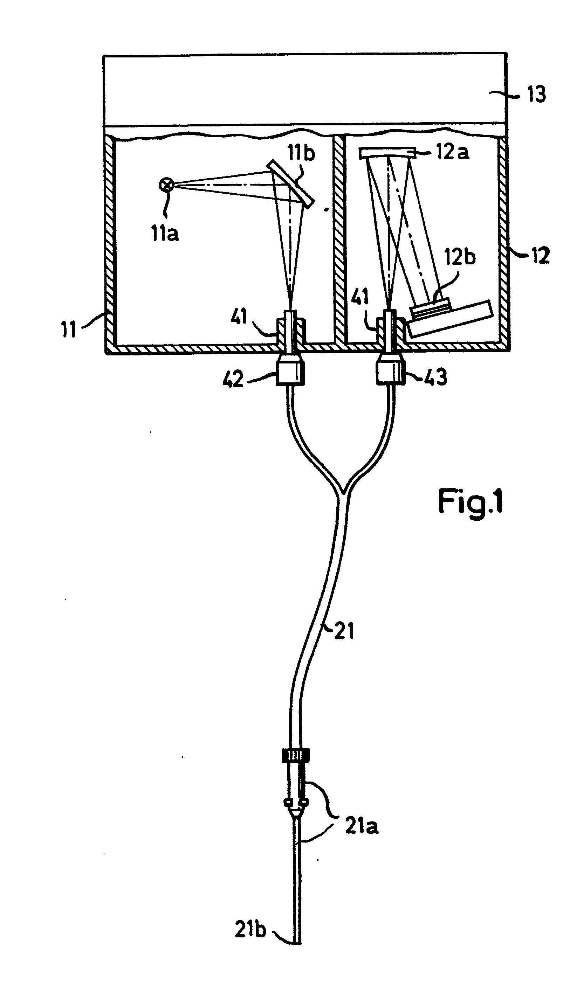

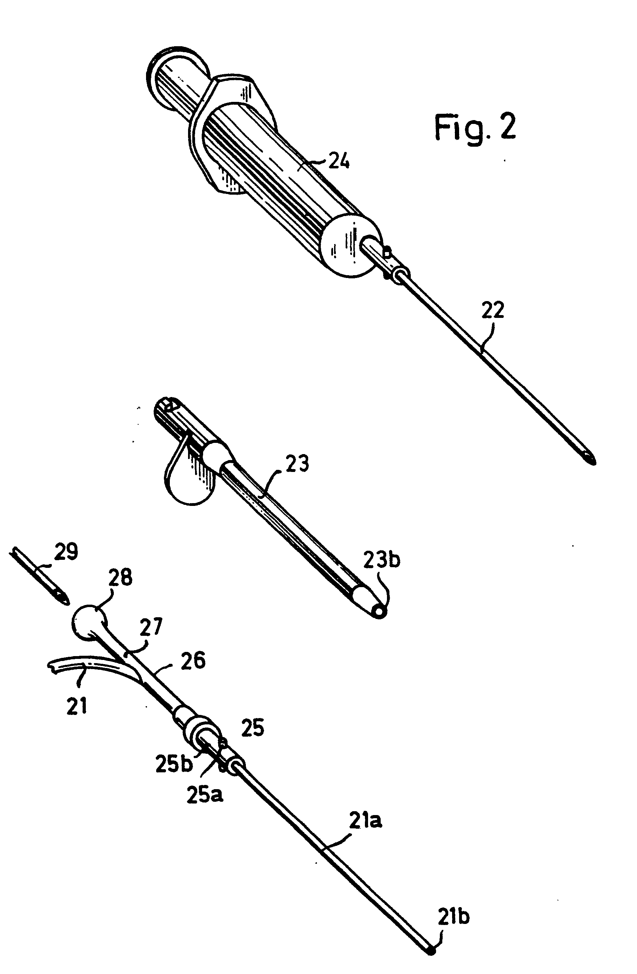

In Fig. 1 ist mit 11 eine Beleuchtungseinrichtung, mit 12 ein Spektrometer und mit 13 das Netzteil und die Elektronik für Beleuchtungseinrichtung und Spektrometer bezeichnet. Die Beleuchtungseinrichtung 11 besteht aus der Xenonlampe 11a, einem elliptischen Hohlspiegel 11b und einem Fassungsteil 41 für die Steckverbindung 42 der Lichtleitersonde 21. Die zweite Steckverbindung 43 der Lichtleitersonde 21 sitzt im Fassungsteil 41 des Spektrometers 12, das ausserdem ein Konkavgitter 12a und eine Diodenzeile 12b enthält. Der Messkopf 21a der Lichtleitersonde 21 ist in Fig. 2 im grösseren Massstab dargestellt. Er kann an Stelle der Kanüle 22 in den Verweilkatheter 23 eingeführt werden, nachdem dieses zusammen mit der Kanüle 22 und gegebenenfalls mit der Injektionsspritze 24 in eine periphere Vene oder Arterie eingeführt worden ist.

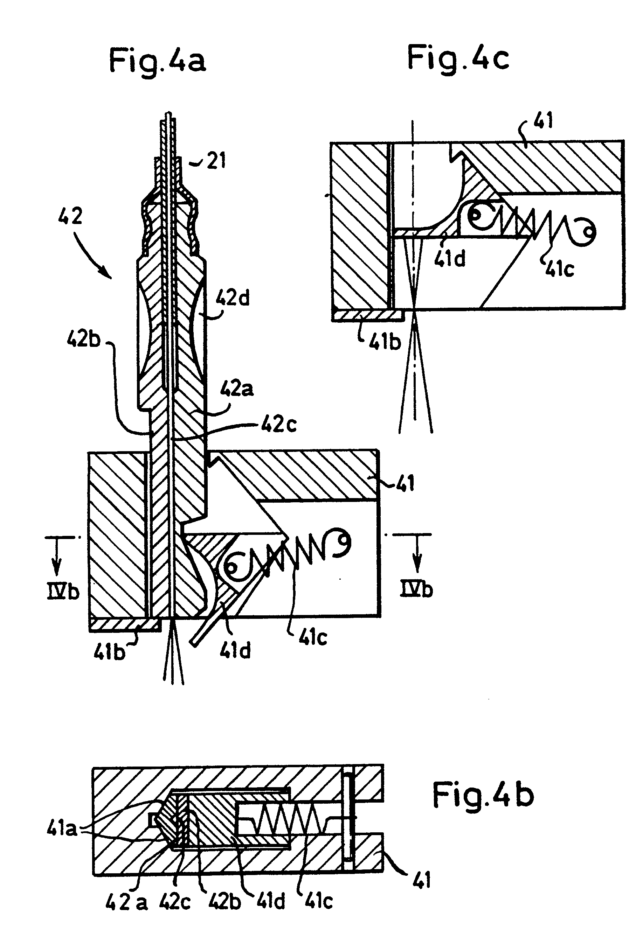

Der Messkopf 21a besteht aus einer dünnwandigen Kanüle, welche die einzelnen Lichtleitfasern, aus denen der Lichtleiter besteht, umgibt. Figur 3 zeigt eine Draufsicht auf die Spitze 21b des Messkopfes von 21a. Die mit 1 bezeichneten Lichtleitfasern kommen von der Beleuchtungseinrichtung 11; die mit 2 bezeichneten Lichtleitfasern gehen zum Spektrometer 12. Die einzelnen Lichtleitfasern sind in bekannter Weise von einem inerten Kunststoff, z.B. Teflon®, ummantelt, der in einem Polymerasationsprozess aufgebracht wird. Diese Ummantelung verbessert die Bruchfestigkeit der Lichtleitfasern wesentlich, verhindert die Blutgerinnung und bewirkt den Zusammenhalt des in Fig. 3 als Ausführungsbeispiel gezeigten dreiecksförmigen Querschnittes des Lichtleiters. Die Zwischenräume 32 zwischen diesem dreiecksförmigen Querschnitt und der Kanülenwand 31 können für den Durchfluss einer Spülflüssigkeit verwendet werden, mit der verhindert wird, dass sich an der Spitze des Messkopfes Blutkörperchen festsetzen.

Die einzelnen Lichtleitfasern 1 bzw. 2 haben einen Durchmesser von z.B. 50 μm. Für die Anordnung der zum Spektrometer 12 gehenden, mit 2 bezeichneten Lichtleitfasern hat sich eine statistische Verteilung innerhalb der übrigen Lichtleitfasern als ausreichend erwiesen.

Am Ende des Messkopfes 21a sitzt als Übergang zu dem weitergehenden flexiblen Lichtleiter 21 des Befestigungsteils 25, welches mit einer Verriegelung 25a und einem leicht konischen Teil 25b im Verweilkatheter 23 eine zuverlässige und dichte Befestigung ermöglicht. Dabei schliesst die Spitze 21b des Messkopfes mit der vorderen Spitze 23b des Verweilkatheters bündig ab.

Nach dem Befestigungsteil 25 hat die äussere Umhüllung des Lichtleiters eine Verzweigung 26 mit welcher die in Fig. 3 dargestellten Kanäle 32 für die Spülflüssigkeit in einen Schlauchteil 27 abgezweigt werden. Dieser Schlauchteil schliesst mit einer durchstechbaren Membran 28 ab, die in bekannter Weise von der Injektionsnadel 29 zum Einbringen der Spülflüssigkeit durchstochen werden kann.

Die Teilung des Lichtleiters 21 in die beiden Zweige für Beleuchtungseinrichtung 11 und Spektrometer 12 erfolgt zweckmässigerweise erst dicht vor diesen Geräten, damit praktisch nur eine Verbindung zwischen Messkopf 21a und den Geräten besteht. In einer vorteilhaften Ausführungsform sind die Lichtleitfasern mit einem Schrumpfschlauch ummantelt, der z.B. beim Sterilisieren aufgeschrumpft wird.

Fig. 4 zeigt die in das Fassungsteil 41 eingeführte Steckverbindung 42 der Lichtleitersonde 21 im vergrösserten Massstab. Dabei ist in Fig. 4a ein Schnitt in der Ebene der optischen Achse der Lichtleitersonde 21 und in Fig. 4b ein Schnitt senkrecht dazu, d.h. parallel zur Gehäusewand der Beleuchtungseinrichtung 11 dargestellt. Die Steckverbindung 42 besteht aus zwei Teilen 42a und 42b. Diese können z.B. als Kunststoffspritzgiessteile mit grosser Genauigkeit hergestellt werden. Sie werden nach dem Einlegen der Lichtfasern in die Aussparung 42c zusammengeklebt oder auf andere Weise zusammengehalten. Die Griffmulden 42d erleichtern das Hineinstecken und Herausnehmen aus dem Fassungsteil 41. Dieses Fassungsteil hat eine prismatische Anlagefläche 41a und einen Anschlag 41b, gegen die das Teil 42b der Steckverbindung nach dem Hineinstecken durch die Feder 41c und das drehbar gelagerte Teil 41d gedrückt wird. Auf diese Weise kommen die in der Aussparung 42c angeordneten Lichtfasern immer in eine genau definierte Lage. Wenn die Steckverbindung 42 aus der Fassung 41 herausgezogen wird, klappt das drehbar gelagerte Teil 41d in die in Fig. 4c dargestellte Lage und verschliesst die Beleuchtungseinrichtung, so dass keine Strahlung der Xenonlampe nach aussen dringen kann.

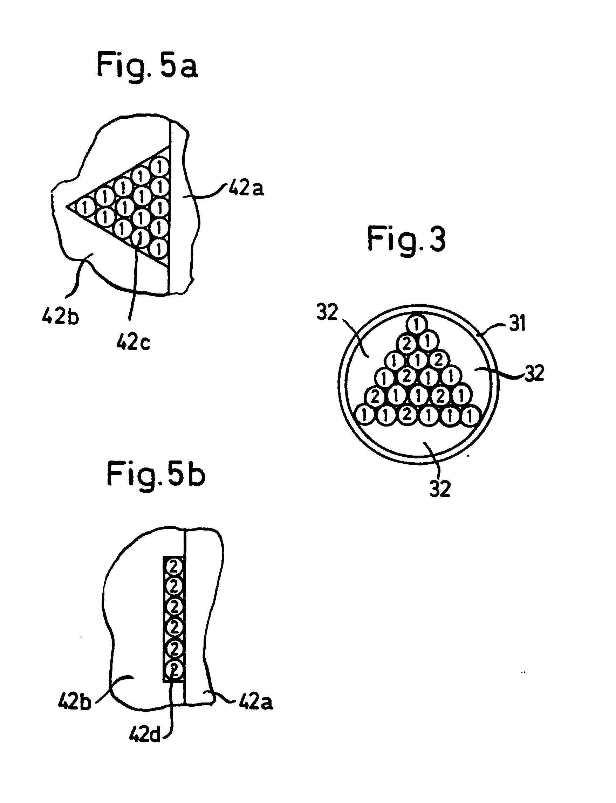

Die Anordnung der Lichtleitfasern an den geräteseitigen Enden der Steckverbindungen 42 bzw. 43 ist in der Fig. 5a für die Beleuchtungseinrichtung 11 und in der Fig. 5b für das Spektrometer 12 dargestellt. Ebenso wie in der Fig. 3 sind die Lichtfasern, die zur Beleuchtungseinrichtung gehen mit 1 und die Lichtfasern, die zum Spektrometer gehen mit 2 bezeichnet. In der Fig. 5a hat das Teil 42b eine dreiecksförmige Aussparung 42c, die so dimensioniert ist, dass z.B. gerade 15 Lichtfasern in dichtester Packung hineingehen.

Auf die Anfangsflächen dieser Lichtfasern wird der Leuchtfleck der Xenonlampe 11a abgebildet, wie dies in den Fig. 1 und 4 dargestellt ist. Durch die dreiecksförmige Anordnung der Lichtfasern wird auf einfache Weise eine gute Ausnutzung der Strahlungsintensität der Lichtquelle erreicht. Selbstverständlich ist auch eine viereckige, sechseckige oder kreisförmige Anordnung der Lichtfasern möglich.

Fig. 5b zeigt die Anordnung der mit 2 bezeichneten Lichtfasern in der Steckverbindung zum Spektrometer. Das Teil 42b hat in diesem Fall eine rechteckförmige Aussparung 42d, die so dimensioniert ist, dass z.B. gerade sechs Lichtfasern 2 hineinpassen. Die Endflächen dieser Lichtfasern bilden den Eintrittsspalt des Spektrometers 12. Ausser diesem derart ausgebildeten Eintrittsspalt besteht das Spektrometer nur noch aus dem holographisch erzeugten Konkavgitter 12a und der Diodenzeile 12b. Ohne irgendwelche weiteren Abbildungsmittel wie Linsen, Spiegel oder ähnlichem wird das Spektrometer direkt von den Lichtfaserenden einerseits vollständig ausgeleuchtet und andererseits die Apertur des Lichtleiters vollständig erfasst. Nur mit einem holographisch erzeugten Beugungsgitter ist eine derartige Anordnung möglich, weil nur mit diesen die wichtigsten Abbildungsfehler (Astigmatismus und Spektrenkrümmung) korrigiert werden können, sofern das Gitter mit der gleichen Geometrie betrieben wird mit der es holographisch erzeugt wurde. Als Beispiel für eine geeignete Dimensionierung des Spektrometers seien folgende Daten genannt: Radius des sphärischen Konkavgitters = 2 × Brennweite 116,3 mm; Durchmesser der freien Öffnung des kreisrunden Gitters 60 mm; das Öffnungsverhältnis des Spektrometers ist damit 1:1,94, das entspricht einem Öffnungwinkel von 30° und damit dem Öffnungswinkel von Lichtleitern.

Die beiden Steckverbindungen 42 und 43 sind zweckmässigerweise so ausgeführt, dass sie unverwechselbar sind, so dass die Anschlüsse an Beleuchtungseinrichtung und Spektrometer nicht vertauscht werden können. Ein wesentlicher Vorteil der dargestellten Ausführungsform der Steckverbindungen ist, dass beim Auswechseln der Lichtleitersonde die Bedienungsperson nur die Lichtleitersonde 21 und die Steckverbindungen 42 und 43 anfassen muss und dadurch mit keinen unsterilen Teile, wie z.B. den Geräten oder den Fassungsteilen 41, in Berührung kommt. Die Lichtleitersonde kann entweder als Wegwerfteil für die Einmalbenutzung oder als leicht sterilisierbares Teil ausgeführt werden.

Für die Messung «in vivo» wird zunächst der Verweilkatheter 23 mit der Injektionsnadel 22 in eine Vene oder Arterie des Patienten eingeführt. Anschliessend wird die Injektionsnadel 22 aus dem Verweilkatheter 23 herausgezogen und der Messkopf 21a in den Verweilkatheter 23 eingeführt. Wenn die Steckverbindungen 42 und 43 in die Fassungen 41 eingesteckt sind, ist das Gerät messbereit. Ein mit der Diodenzeile 12b des Spektrometers 12 verbundener Rechner ermittelt aus der Lage der Maxima die Sauerstoffkonzentration des Blutes und zeigt diese für den Anästhesisten an. Selbstverständlich ist auch eine Registrierung und/oder eine akustische Signalgabe beim Unterschreiten eines kritischen Wertes möglich.

**Patentansprüche**

1. Gerät zur Spektrenmessung mit einem Diodenzeilenspektrometer (12), einer Beleuchtungseinrichtung (11) und einer y-förmigen Lichtleitersonde (21), deren erster Zweig mit der Beleuchtungseinrichtung (11) und deren zweiter Zweig mit dem Diodenzeilenspektrometer (12) verbunden ist, so dass einem Eintrittsspalt des Diodenzeilenspektrometers (12) das vom untersuchten Objekt veränderte Licht zugeführt ist, und deren Stamm in einem Messkopf (21a) endet, dadurch gekennzeichnet, dass ein in eine Blutbahn einführbarer Teil des Messkopfes (21a) der Lichtleitersonde (21) mit einem Durchmesser von maximal 0,5 mm ausgebildet ist, dass jeder der beiden Zweige der Lichtleitersonde (21) durch eine Steckverbindung (42, 43) und ein diese aufnehmendes Fassungsteil (41) mit genau definierter Lage der Lichtleiter mit dem Diodenzeilenspektrometer (12) bzw. mit der Beleuchtungseinrichtung verbunden ist, dass die Endflächen der Lichtfasern (2) in der Steckverbindung (43) zum Diodenzeilenspektrometer (12) den Eintrittsspalt des Diodenzeilenspektrometers (12) bilden, dass das Diodenzeilenspektrometer (12) ein holographisch erzeugtes Beugungsgitter (12a) aufweist und ein Öffnungsverhältnis hat, das die Apertur des Lichtleiters ohne Beschnitt aufnimmt.

2. Gerät nach Anspruch 1, dadurch gekennzeichnet, dass die Lichtleitersonde (21) aus mehreren Lichtleitfasern (1) für die Hinleitung des Lichtes der Beleuchtungseinrichtung zum Messort und mehreren Lichtleitfasern (2) für die Fortleitung des Streulichtes vom Messort besteht und dass die einzelnen Lichtleitfasern von inerten Hüllen zum Schutz gegen Bruch und Blutgerinnung umgeben sind.

3. Gerät nach Anspruch 2, dadurch gekennzeichnet, dass die Lichtleitfasern (2) in der Steckverbindung (43) für das Diodenzeilenspektrometer (12) in Reihe angeordnet sind.

4. Gerät nach Anspruch 2 oder 3, dadurch gekennzeichnet, dass in der Spitze (21b) des Messkopfes (21a) die Lichtleitfasern (1) für die Hinleitung des Lichtes und die Lichtleitfasern (2) für die Fortleitung des Streulichtes durchmischt angeordnet sind.

5. Gerät nach einem der Ansprüche 1 bis 4, dadurch gekennzeichnet, dass die Lichtleiter mit einem bei der Sterilisation aufgeschrumpften Schrumpfschlauch ummantelt sind.

6. Gerät nach einem der Ansprüche 1 bis 5, dadurch gekennzeichnet, dass der Messkopf (21a) der Lichtleitersonde (21) für die Einführung in einen Verweilkatheter (23) ausgebildet ist.

7. Gerät nach einem der Ansprüche 1 bis 6, dadurch gekennzeichnet, dass die Lichtleitersonde (21) mit einem oder mehreren Spülkanälen (32) ausgebildet ist.

8. Gerät nach einem der Ansprüche 1 bis 7, dadurch gekennzeichnet, dass die Lichtleitersonde (21) einschliesslich der Steckverbindungen (42, 43) als sterilisiertes Wegwerfteil für Einmalbenutzung ausgebildet ist.

9. Gerät nach einem der Ansprüche 1 bis 8, dadurch gekennzeichnet, dass die Beleuchtungseinrichtung (11) als Lichtquelle (11a) eine Xenonlampe aufweist.

**Claims**

1. An instrument for spectral measurement with a diode line spectrometer (12), an illuminating device (11) and a y-shaped light guide probe (21), the first branch of which is connected with the illuminating device (11) and the second branch of which is connected with the diode line spectrometer (12), so that the light which is altered by the probe is supplied to an entrance slit of the diode line spectrometer (12), and the trunk of which ends in a measuring head (21a), characterized by the fact, that a part of the measuring head (21a) which is introduced into a bloodstream is developed with a diameter of not more than 0,5 mm, that every one of the two branches of the light guide probe (21) is connected to the diode line spectrometer (12) resp. to the illuminating device (11) by a plug connection (42, 43) and a socket part (41) accomodating this with precisely defined position of the light guides, that the end surfaces of the light guide fibers (2) in the plug connection (43) to the diode line spectrometer (12) are constituting the entrance slit of the diode line spectrometer (12), that the diode line spectrometer (12) comprises an holographic produced grating (12a) and has an aperture ratio, which receives the aperture of the light guide without trimming.

2. An instrument according to claim 1, characterized by the fact that the light-guide probe (21) consists of a plurality of light-guide fibers (1) for the feeding of the light of the illuminating device to the place of measurement and of a plurality of light-guide fibers (2) for conducting the scattered light from the place of measurement and that the light-guide fibers are surrounded by inert sleeves in order to protect them against breakage and coagulation of the blood.

3. An instrument according to claim 2, characterized by the fact that the light-guide fibers (2) are arranged in row in the plug connection (43) for the diode line spectrometer (12).

4. An instrument according to claim 2 or 3, characterized by the fact that at the top (21b) of the measuring head (21a) the light-guide fibers (1) for feeding the light and the light guide fibres (2) for conducting the scattered light are arranged mixed together.

5. An instrument according to any of claims 1 to 4, characterized by the fact that the light guides are surrounded by a shrink tube which is shrunk-on upon the sterilization.

6. An instrument according to any of claims 1 to 5, characterized by the fact that the measuring head (21b) of the light-guide probe (21) is developed for introduction into an indwelling catheter (23).

7. An instrument according to any of claims 1 to 6, characterized by the fact that the light-guide probe (21) is developed with one or more wash channels (32).

8. An instrument according to any of claims 1 to 7, characterized by the fact that the light-guide probe (21) including the plugs (42, 43) is developed as a sterilizable disposable part for one-time use.

9. An instrument according to any of claims 1 to 8, characterized by the fact that the illuminating device (11) contains a xenon lamp as source of light (11a).

**Revendications**

1. Appareil pour la mesure de spectres, comprenant un spectromètre à rangée de diodes (12), un dispositif d'éclairage (11) et une sonde conductrice de lumière en Y (21) dont une première branche est reliée au dispositif d'éclairage (11), dont la seconde branche est reliée au spectromètre (12), de manière que la lumière, modifiée par l'objet examinée, soit amenée à une fente d'entrée du spectromètre (12), et dont la tige se termine dans une tête de mesure (21a), caractérisé en ce que la tête de mesure (21a) de la sonde conductrice de lumière (21) est pourvue d'une partie pouvant être introduite dans une voie sanguine et ayant un diamètre de tout au plus 0,5 mm, que chacune des deux branches de la sonde conductrice de lumière (21) est reliée, l'une au spectromètre à rangée de diodes (12) et l'autre au dispositif d'éclairage, par une connexion à enfichage (42, 43) et par une partie formant une douille (41) pour sa réception, avec définition exacte de la position du conducteur de lumière, que les faces terminales des fibres conductrices de lumière (2) dans la connexion du spectromètre (12) forment la fente d'entrée de celui-ci et que le spectromètre à rangée de diodes (12) présente un réseau de diffraction (12a) produit par holographie et possède un taux d'ouverture qui reçoit l'ouverture du conducteur de lumière, sans qu'il soit nécessaire de le couper sur les côtés.

2. Appareil selon la revendication 1, caractérisé en ce que la sonde conductrice de lumière (21) est constituée de plusieurs fibres conductrices de lumière (1) pour amener la lumière du dispositif d'éclairage au point de mesure et de plusieurs fibres conductrices de lumière (2) pour évacuer la lumière dispersée à partir du point de mesure, et que les différentes fibres sont enrobées de gaines inertes pour la protection contre la rupture et la coagulation du sang.

3. Appareil selon la revendication 2, caractérisé en ce que les fibres conductrices de lumière (2) pour évacuer la lumière depuis le point de mesure, sont disposées en ligne dans la connexion à enfichage (43) pour le spectromètre à rangée de diodes (12).

4. Appareil selon la revendication 2 ou 3, caractérisé en ce que les fibres (1) pour l'amenée de la lumière et les fibres (2) pour l'évacuation de la lumière dispersée sont disposées dans la pointe (21b) de la tête de mesure (21a) en étant mélangées.

5. Appareil selon une des revendications 1 à 4, caractérisé en ce que les conducteurs de lumière sont enveloppés d'un tuyau rétractable qui est rétracté sur les conducteurs de lumière lors de la stérilisation.

6. Appareil selon une des revendications 1 à 5, caractérisé en ce que la tête de mesure (21a) de la sonde conductrice de lumière (21) est conçue pour être introduite dans un cathéter à demeure (23).

7. Appareil selon une des revendications 1 à 6, caractérisé en ce que la sonde conductrice de lumière (21) est pourvue d'un ou plusieurs canaux de rinçage (32).

8. Appareil selon une des revendicatios 1 à 7, caractérisé en ce que la sonde conductrice de lumière (21), y compris les connexions à enfichage (42, 43), est réalisée comme une pièce stérilisée à jeter après un emploi unique.

9. Appareil selon une des revendications 1 à 8, caractérisé en ce que le dispositif d'éclairage (11) contient une lampe à xénon en tant que source de lumière (11a).

Fig.1

Fig. 2

Fig.4a

Fig.4c

Fig.4b

# Fig. 5a

42a

42b

42c

# Fig. 3

32

31

32

32

# Fig. 5b

42b

42a

42d